# EUROPEAN PATENT SPECIFICATION

(11) **EP 2 244 690 B1**
(45) Date of publication and mention of the grant of the patent: **02.08.2017**
(21) Application number: 08837020.0
(22) Date of filing: 10.10.2008
(51) Int. Cl.: A61K 8/30, A61P 17/00, A61P 17/02, A61P 17/06, A61P 17/10, A61P 17/14, A61K 31/085

(54) **Topical compositions for treating inflammatory disorders, diseases and conditions**
Topische Zusammensetzungen zur Behandlungen von entzündlichen Erkrankungen und Leiden
Compositions topiques utilisées pour traiter des maladies et affections inflammatoires

(30) Priority: 10.10.2007 US 998345 P; 05.05.2008 US 126478
(43) Date of publication of application: 03.11.2010
(60) Divisional application: 17174953.4
(73) Proprietor: Therametics, Llc, Oklahoma City, OK 73104 (US)
(72) Inventor: FULLER, Bryan, B., Edmond OK 73034 (US)
(74) Representative: Stuttard, Garry Philip
(86) International application number: PCT/US2008/079534
(87) International publication number: WO 2009/049172

(56) References cited:
- WO-A1-03/043621
- WO-A1-2004/000305
- US-A1- 2006 216 251
- US-A1- 2006 222 671
- US-A1- 2007 207 220
- MURAKAMI Y ET AL: "Dehydrodiisoeugenol, an isoeugenol dimer, inhibits lipopolysaccharide-stimulated nuclear factor kappa B activation and cyclooxygenase-2 expression in macrophages", ARCHIVES OF BIOCHEMISTRY AND BIOPHYSICS, ACADEMIC PRESS, US, vol. 434, no. 2, 15 February 2005 (2005-02-15), pages 326-332, XP004705012, ISSN: 0003-9861, DOI: DOI:10.1016/J.ABB.2004.11.013
- European Commission: "Opinion on Fragrance allergens in cosmetic products", 13 December 2011 (2011-12-13)
- National Toxicology Program: "Executive summary of safety and toxicity information - isoeugenol", 10 April 1991 (1991-04-10)
- Declaration by Amber J. Howerton dated 28.03.2016
- "5-Hydroxymethyl-2-furaldeyde" In: "Material Safety Data Sheet", 2009
- "Furfural" In: "Saftey Data Sheet", 2003
- "Abstract for TR-482-Furfuryl Alcohol (CASRN 98-00-0)" In: "National Toxicology Program", 1999

## Description

The present invention relates to transdermal compositions for use in treating an inflammatory skin disorder, condition or disease comprising dihydroeugenol or a salt thereof.

Human skin comprises an epidermis layer which is predominantly composed of keratinocytes and a small number of melanocytes and Langerhans cells (antigen presenting cells) and a dermis layer which is primarily composed of fibroblasts. The majority of skin disorders involve inflammation triggered by some insult to the skin. Keratinocytes respond quickly to environmental stimuli (e.g., UV radiation (UVR), allergens, irritants or physical damage) by producing a variety of inflammatory mediators, including cytokines (e.g., IL-1, TNF-alpha, and IL-6) and chemokines (e.g., IL-8). One of the most active inflammatory mediators is PGE-2 (Prostaglandin E2) and, of course, many topical dermatology drugs have been designed to lower levels of PGE-2. The fibroblasts in the dermis also produce PGE-2 along with a variety of chemokines, cytokines and matrix destroying enzymes such as collagenase (MMP-1).

The identification of compounds that can suppress the production of inflammatory mediators in the skin would allow effective topical products to be developed to treat a variety of inflammatory skin diseases or disorders including eczema, radiation dermatitis, atopic dermatitis, actinic keratosis, seborrheic dermatitis, other dermatitic diseases and acne. Compositions able to treat other dermatological conditions such as aging effects and hyperpigmentation would also be desirable.

WO-A-2004/000305 discloses ointments comprising isoeugenol for topical administration to treat psoriasis, hair growth and eczema.

An aspect of the present invention provides transdermal composition for use in the treatment of an inflammatory skin disorder, disease, or condition in a subject, the topical composition comprising:
an active agent comprising dihydroeugenol or a salt thereof, wherein the active agent is present in the range about 0.1% to about 10% by weight of the transdermal composition; and the remainder of the transdermal composition being an aqueous, aqueous mixed or non aqueous, pharmaceutically-acceptable carrier or vehicle in which the active agent is suspended or dissolved, wherein the pharmaceutically acceptable carrier or vehicle is effective in carrying or enabling passage of the active agent into the epidermis or dermis of the skin of the subject where the active agent has its effect on the inflammatory skin disorder, disease or condition and wherein the inflammatory skin disorder, disease, or condition of the subject is at least one of inflammation, rosacea, allergic contact dermatitis, irritant contact dermatitis, seborrheic dermatitis, radiation dermatitis, erythema, psoriasis, atopic dermatitis, eczemas, actinic keratosis, acne, scarring, aging and alopecia areata.

The pharmaceutically-acceptable carrier or vehicle can comprise a penetration enhancer.

The pharmaceutically-acceptable carrier or vehicle can be non-aqueous and can be absent emulsifiers.

The carrier can primarily comprise at least 25% by weight of a silicone.

The topical composition can further comprise cinnamaldehyde.

The topical composition can be effective in percutaneous transmission such that the active agent can be maintained in the epidermis or dermis at a concentration in a range of from 10 µM to 500 µM.

The topical composition can stimulate dermal production of collagen, elastin and TIMPs and inhibits production of MMPs.

The dermatological condition may be rosacea or atopic dermatitis.

The active agent can further comprise isoeugenol or a salt, ester or ether thereof.

The dermatological condition may be radiation dermatitis and/or erythema.

The dihydroeugenol can be 0.5% to 5% by weight of the topical composition.

The dihydroeugenol can be 1% to 4% by weight of the topical composition.

The active agent can be dihydroeugenol, isoeugenol or a salt or ester thereof and cinnamaldehyde, wherein the dermatological condition is acne.

The active agent can comprise dihydroeugenol or a salt thereof and isoeugenol or a salt, ester or ether thereof, wherein the dermatological condition is psoriasis.

The active agent can be maintained in the epidermis or dermis of the skin at a concentration in a range of from 10 µM to 500 µM.
Figure 1 is a graph showing the effects of dihydroeugenol (DHE) on UV-induced PGE-2 in fibroblasts.
Figure 2 is a graph showing the effects of DHE on UV-induced PGE-2 in keratinocytes.
Figure 3 is a graph showing the effects of DHE on TPA-induced TNF-α in keratinocytes.
Figure 4 is a graph showing the effects of DHE on LPS-induced inflammatory cytokines in monocytes.
Figure 5 is a photograph of an arm of a human subject which was exposed to erythema-inducing UVB radiation and treated with a lotion of the present invention.
Figure 6 shows photographs of a human subject's face which has symptoms of rosacea. Facial areas of rosacea are shown in (A) while these areas, 12 weeks after initiation of treatment with DHE, IE, and cinnamaldehyde, are shown in (B).
Figure 7 shows the effects of topical DHE application on patients undergoing radiation treatment. (A) Radiation dermatitis in a typical radiation patient untreated for radiation dermatitis. (B) A patient was treated with a topical 2% DHE lotion daily during 35 radiation treatments. The photograph was taken after the 35^{th} treatment and the skin shows no evidence of radiation dermatitis. (C) A close-up photograph of the patient of (B), one month after the end of radiation treatments. There is no evidence of radiation damage to the skin.
Figure 8 is a graph showing stimulation of collagen 1 mRNA in fibroblasts by DHE, isoeugenol (IE), and Ethyl vanillin (EV).
Figure 9 is a graph showing stimulation of elastin mRNA in fibroblasts by DHE, IE and EV.
Figure 10 is a graph showing stimulation of hyaluronic acid synthase-2 in RNA in fibroblasts by DHE, IE and EV.
Figure 11 is a graph showing stimulation of tissue inhibitor of metalloprotinease-1 mRNA (TIMP-1) in fibroblasts by DHE, IE and EV.
Figure 12 is a graph showing inhibition of matrix metalloprotenases (MMPs) in fibroblasts by ethyl vanillin.
Figure 13 is a graph showing inhibition of melanin synthesis *in vitro* by isoeugenol (referred to in the figure as TH-212).
Figure 14 shows photographs which demonstrate the loss of pigment in human melanocyte cultures treated with isoeugenol (TH-212) and isoeugenol acetate (referred to in the figure as TH-213) for 9 days.
Figure 15 shows photographs which demonstrate how a topically-applied formulation of isoeugenol acetate (referred to in the figure as TH-212 ester) plus salicylic acid causes a clearing of acne and hyperpigmentation after a 4 week treatment.
Figure 16 shows photographs which demonstrate how a topically-applied formulation of isoeugenol acetate (TH-212 ester) plus 2% salicylic acid removes hyperpigmentation after a 30-day treatment period.
Figure 17 shows photographs which demonstrate how a topically-applied formulation of isoeugenol acetate (TH-212 ester) plus 2% salicylic acid reduces sun-induced hyperpigmentation after a 30-day treatment period.
Figure 18 shows a photograph of cell extracts treated with isoeugenol (TH-212) and isoeugenol acetate (TH-213) then provided with L-DOPA, a tyrosinase substrate.
Figure 19 shows photographs which demonstrate psoriatic skin before (A) and after (B) treatment with a formulation comprising DHE and IE.

The present invention relates to topical compositions for use in treating a variety of skin disorders, diseases and conditions including rosacea, radiation dermatitis, erythemas (sunburns), psoriasis, atopic dermatitis, allergic and irritant contact dermatitis, actinic keratosis, acne, scarring, seborrheic dermatitis, eczema and alopecia areata. The topical compositions act (for example) on skin keratinocytes and fibroblasts and on immune cells such as monocytes and on melanocytes.

Inflammatory skin diseases are the most common problem in dermatology. They come in many forms, from occasional rashes accompanied by itching and redness to chronic conditions such as dermatitis (eczema), rosacea, seborrheic dermatitis and psoriasis. However they are all linked by one common factor, inflammation. It has been found that the inflammatory markers (cytokines) produced by skin and immune cells are required for the development of an inflammatory response, such as atopic dermatitis and radiation dermatitis. The topical compositions used in the present invention comprise agents that suppress the production of a variety of inflammatory responses in cultured skin cells (keratinocytes and fibroblasts) and immune cells (monocytes and T-lymphocytes) and in intact living skin. As a result of blocking these inflammatory processes in the skin, the agents are able to effectively reduce or eliminate a variety of inflammatory symptoms that occur with common skin problems.

Rosacea is a vascular, inflammatory skin disorder that affects approximately 5% of the population and is characterized by frequent periods of facial redness or flushing caused by over-active capillaries. Over time, this chronic state of skin inflammation gives rise to a variety of rosacea symptoms. Rosacea is sometimes characterized mistakenly as adult-acne because patients present with a reddened face and acne-like symptoms. However, individuals affected with this skin disease also may have persistent redness with accompanying pain and itching in areas such as the forehead, chin, nose, ears, chest and back. As the disease progresses, small blood vessels and tiny pimples (called papules or pustules) begin to appear on and around the reddened area. In severe cases rosacea can affect the eyes (ocular rosacea) and cause disfigurement of the nose (rhynophyma). In addition to the physical symptoms associated with rosacea, patients also suffer significant psychological and social problems if left untreated.

Research shows that there are two distinct types of skin aging. Aging caused by the genes we inherit is called *intrinsic* (internal) *aging.* The other type of aging is known as *extrinsic* (external) *aging* and is caused by environmental factors, such as exposure to the sun's rays. Intrinsic aging, also known as the natural aging process, is a continuous process that normally begins in our mid-20s. Within the skin the dermis of normal (wrinkle-free) skin is composed of abundant amounts of type I collagen and type VII collagen, as well as elastin, which provides tissue strength, resiliency and recoil. However, as the dermal fibroblasts which produce collagen and elastin begin to age, they produce decreasing amounts of these proteins. Further, aging fibroblasts produce increased amounts of enzymes called matrix metaloproteinases (MMPs) which degrade collagen and elastin. This results in a drastic loss of collagen and elastin over time and results in skin laxity and fragility, visible in the form of fine lines and wrinkles. The signs of intrinsic aging are: fine wrinkles, thin and transparent skin, loss of underlying fat, leading to hollowed cheeks and eye sockets as well as noticeable loss of firmness on the hands and neck, dry skin that may itch, graying hair that eventually turns white, and hair loss. Genes control how quickly the normal aging process unfolds. Some persons notice their first gray hairs in their 20s; others do not see graying until their 40s.

A number of extrinsic aging factors often act together with the normal aging process to prematurely age our skin. Most premature aging is caused by sun exposure. Other external factors that prematurely age our skin are repetitive facial expressions, gravity and smoking. Without protection from the sun's rays, just a few minutes of exposure each day over the years can cause noticeable changes to the skin. Freckles, age spots, spider veins on the face, rough and leathery skin, fine wrinkles that disappear when stretched, loose skin, a blotchy complexion and skin cancer can all be traced to sun exposure. "Photoaging" is the term dermatologists use to describe this type of aging caused by exposure to the sun's rays. The amount of photoaging that develops depends on: (1) a person's skin color and (2) his or her history of long-term or intense sun exposure. Persons with fair skin who have a history of sun exposure develop more signs of photoaging than those with dark skin. In the darkest skin, the signs of photoaging are usually limited to fine wrinkles and a mottled complexion.

Photoaging occurs over a period of years. With repeated exposure to the sun, the skin loses the ability to repair itself and the damage accumulates. Scientific studies have shown that repeated ultraviolet (UV) exposure impairs the synthesis of new collagen and increases the expression of MMP enzymes which break down collagen. The sun also causes excessive elastin production but the elastin made is abnormal and aggregates into clumps, leading to a condition referred to by dermatologists as elastosis. Due to the loss of collagen and the production of abnormal elastin, sun-weakened skin ceases to spring back compared to skin protected from UV rays. Skin also becomes loose, wrinkled and leathery much earlier with unprotected exposure to sunlight.

While there is nothing currently available to stop the aging process, it is possible to slow the rate at which the skin ages. As described above, both intrinsic and photoaging are due to the breakdown and loss of collagen and elastin in the skin. The present invention is a topical composition for use which contains an agent that has been scientifically proven herein to increase the production of collagen and elastin as well as reduce the expression of MMP enzymes. These two effects slow the aging process and can even aid in rebuilding the dermal matrix which reduces the appearance of new and existing wrinkles and fine lines.

Acne is the most common skin disorder seen by doctors and affects almost everyone at some time. Teenagers are affected most often. Acne can cause a great deal of embarrassment and anxiety and can even cause people to become depressed which can lead to withdrawing from friends, and performing poorly at school or work. The exact cause of acne is unknown, but the following factors are considered important. (1) Acne is the visible end result of hormonal, bacterial and inflammatory disturbances that take place at the level of the oil pore (pilosebaceous follicle). (2) As the process advances, greater amounts of oil may be produced within the sebaceous glands, though the change in composition and quality of the oil may be more important than the quantity, the scale produced on the inside walls of the hair follicle becomes stickier and it builds up and blocks the pore which shows up as whiteheads and blackheads (comedones). (3) The acne bacteria (*Propionobacterium acnes*) grow and multiply in the retained oil. The sebum acts as a nutrition source for the bacterial, which in turn release chemicals within the pore. These alert and attract white cells from the blood leading to inflammation. (4) As these inflamed hair follicles (pores) and glands enlarge, the surrounding skin also becomes inflamed and may lead to even larger lumps and cysts (also called nodules). (5) Inflammation may damage the cells that make collagen. Less collagen production causes thinning of the skin, which is seen as depressed scars. Occasionally, collagen production will increase, which then causes the scars to become thickened. The present topical compositions may be used to heal scars from acne and stretch marks by improving their structure and coloration.

Psoriasis is a chronic (long-lasting) skin disease characterized by scaling and inflammation. Scaling occurs when cells in the outer layer of skin reproduce faster than normal and pile up on the skin's surface. Psoriasis affects 2 to 2.6 percent of the United States population (almost 5.8 to 7 million people). It occurs in all age groups and about equally in men and women. People with psoriasis may suffer discomfort, restricted motion of joints and emotional distress. When psoriasis develops, patches of skin thicken, redden and become covered with silvery scales. These patches are sometimes referred to as plaques. They may itch or burn. The skin at joints may crack. Psoriasis most often occurs on the elbows, knees, scalp, lower back, face, palms and soles of the feet. The disease also may affect the fingernails, toenails and the soft tissues inside the mouth and genitalia. About 10 percent of people with psoriasis have joint inflammation that produces symptoms of arthritis. This condition is called psoriatic arthritis.

Research indicates that psoriasis may be a disorder of the immune system. The immune system includes a type of white blood cell, called a T cell, that normally helps protect the body against infection and disease. In psoriasis, the immune system produces too many T cells in the skin. These T cells trigger the inflammation and excessive skin cell reproduction seen in people with psoriasis. This leads to inflammation and flaking of skin. The present topical composition inhibits the production of the same inflammatory mediators that other psoriasis therapies target. Since it is in topical form, it does not require frequent injections nor does it lower body's overall immune function.

Eczema is a general term for many types of skin inflammation (dermatitis). Atopic dermatitis is the most common of the many types of eczema. Several other forms have very similar symptoms. The diverse types of eczema are listed and briefly described below.
(1) Atopic dermatitis is a chronic skin disease characterized by itchy, inflamed skin. The word "dermatitis" means inflammation of the skin. "Atopic" refers to diseases that are hereditary, tend to run in families and often occur together. These diseases include asthma, hay fever and atopic dermatitis. In atopic dermatitis, the skin becomes extremely itchy and inflamed, causing redness, swelling, cracking, weeping, crusting and scaling. Atopic dermatitis most often affects infants and young children, but it can continue into adulthood or first show up later in life. In most cases, there are periods of time when the disease is worse (called exacerbations or flares) which are followed by periods when the skin improves or clears up entirely (called remissions). Many children with atopic dermatitis enter into a permanent remission of the disease when they get older, although their skin often remains dry and easily irritated. Environmental factors can activate symptoms of atopic dermatitis at any time in the lives of individuals who have inherited the atopic disease trait. The cause of atopic dermatitis is unknown, but the disease seems to result from a combination of genetic and environmental factors. Evidence suggests that the disease is associated with other so-called atopic disorders such as hay fever and asthma which many people with atopic dermatitis also have. In addition, many children who outgrow the symptoms of atopic dermatitis go on to develop hay fever or asthma. Although one disorder does not cause another, they may be related thereby giving researchers clues to understanding atopic dermatitis. Atopic dermatitis is very common and affects males and females equally and accounts for 10 to 20 % of all referrals to dermatologists. Atopic dermatitis occurs most often in infants and children and its onset decreases substantially with age. Scientists estimate that 65 percent of patients develop symptoms in the first year of life, and 90 percent develop symptoms before the age of 5. Onset after age 30 is less common and often occurs after exposure of the skin to harsh conditions. People who live in urban areas and in climates with low humidity seem to be at an increased risk for developing atopic dermatitis. About 10% of all infants and young children experience symptoms of the disease. Roughly 60 percent of these infants continue to have one or more symptoms of atopic dermatitis even after they reach adulthood. This means that more than 15 million people in the United States have symptoms of the disease.
(2) Contact eczema is a localized reaction that includes redness, itching, and burning where the skin has come into contact with an allergen (an allergy-causing substance) or with an irritant such as an acid, a detergent (soap, bodywash) or other chemical.
(3) Allergic contact eczema is a red, itchy, weepy reaction where the skin has come into contact with a substance that the immune system recognizes as foreign, such as poison ivy or certain preservatives in creams and lotions.
(4) Seborrheic eczema is a form of skin inflammation of unknown cause but which is associated with a certain type of yeast that lives on the skin. Seborrheic eczema presents as yellowish, oily, scaly patches of skin on the scalp, face and occasionally other parts of the body (called cradle cap in infants).
(5) Nummular eczema is coin-shaped patches of irritated skin-most commonly on the arms, back, buttocks and lower legs-that may be crusted, scaling and extremely itchy.
(6) Neurodermatitis is scaly patches of skin on the head, lower legs, wrists or forearms caused by a localized itch (such as an insect bite) that becomes intensely irritated when scratched.
(7) Stasis dermatitis is a skin irritation on the lower legs, generally related to circulatory problems.
(8) Dyshidrotic eczema is irritation of the skin on the palms of hands and soles of the feet characterized by clear, deep blisters that itch and burn.

Radiation therapy is another skin disorder that can have some unpleasant side effects in the skin. The following are the most common acute and chronic side effects resulting from radiation. Unforeseen side effects may occur because of the unique and varied tolerance of individual persons. Late effects of treatment may not always be predictable and may be influenced by concurrent and/or subsequent treatment for this and other diseases.

Specific side effects of radiotherapy depend on the part of the body being treated as well as the dose given. In general, the first change is a reddening of the skin, resembling a sunburn. In many patients this is all that is experienced. However, in most patients the burn can be severe and in many cases equivalent to second degree burns. Like a sunburn, the involved area is often sensitive and even painful to the touch. In addition, the overlying skin may break down and the area may remain open until several days to weeks after the course of radiation is completed. Once the course of radiotherapy is completed, the redness will gradually go away and any open areas normally will heal. However the skin in this area will most likely develop features of aged skin including pronounced wrinkling, skin thinning, stiffness and/or dryness, as well as possible pigmentation changes.

Most of the current treatment options for radiation dermatitis involve the use of emollients or aloe gels in an attempt to keep the skin moisturized. However as those who have had the experience of sunburn know, moisturization helps the skin from drying out but does not reduce the pain or redness which are caused by inflammation. The present DHE-containing topical compositions may be a moisturizing lotion that contains the active agent in the form of a bioactive that is able to reduce skin redness and pain associated with radiation therapy.

The DHE-containing topical composition may be administered to a subject having rosacea for example for treating and reducing the rosacea on the subject's skin. The DHE-containing topical composition may also be used for example to treat and/or inhibit psoriasis and radiation dermatitis in a subject. The DHE-containing topical composition may further comprise one or more of isoeugenol (IE) or a salt, ester or ether thereof (for example isoeugenyl acetate or methyl isoeugenol). These DHE-containing topical compositions may also be used to treat inflammatory skin conditions such as actinic keratosis, acne, scarring, allergic and irritant contact dermatitis, atopic dermatitis, erythema (sunburn), hand eczema, seborrheic dermatitis or alopecia areata.

In preferred embodiments, the DHE-containing topical compositions comprise synthetic and/or natural versions of dihydroeugenol (DHE) or a salt thereof (and optionally isoeugenol (IE), ethyl vanillin (EV) or salts, esters, ethers or derivatives thereof).

An optional component of the DHE-containing composition is cinnamaldehyde at a concentration which preferably is in the range of 0.01% to 5%. Cinnamaldehyde is preferably included in the DHE-containing composition for use in treatment of rosacea, acne and seborrheic dermatitis. The cinnamaldehyde provides both anti-inflammatory and anti-bacterial/anti-microbial effects against (for example) bacteria which contribute to acne and yeasts which contribute to seborrheic dermatitis.

### Definitions

Certain terms used herein have the following meanings:
"Ionizing radiation" refers to any radiation that ionizes atoms or molecules of matter. It may consist of particles (such as electrons) or it may be electromagnetic (ultraviolet radiation; X-rays; gamma radiation). Ionizing radiation occurs naturally (for example as a component of sunlight) and is emitted by radioactive substances. It is also produced (for example) artificially in X-ray machines, particle accelerators and nuclear reactors.

"Isolated" used to define the state of purity of synthetic or natural compounds used in the invention means substantially freed of (*ie* at least about 90% and especially at least about 95%) or separated from related feedstocks, raw materials, co-products or (in the case of naturally-occurring mixtures) related materials with which the compound appears in nature.

"Pharmaceutically-acceptable topical carrier" and equivalent terms refer to an inactive liquid or cream vehicle capable of suspending or dissolving compounds described herein and/or salts, esters, ethers, or derivatives thereof or combinations thereof and having the properties of being generally nontoxic and noninflammatory when applied to the skin. This term is specifically intended to encompass carrier materials approved for use in topical cosmetics and topical pharmaceuticals. Representative carriers include water, silicone fluids, vegetable oils, mineral oils, cream bases, lotion bases, ointment bases and the like. These bases include suspending agents, thickeners, penetration enhancers and the like. Their formulation is well known to those in the art of cosmetics and topical pharmaceuticals. Additional information concerning carriers can be found in Remington's Pharmaceutical Sciences, 20th edition, 2000, Lippincott, Williams and Wilkins.

"Therapeutically effective dose" means a dose which when applied topically to the skin of a patient afflicted with a dermatologic or other cosmetic or medical disease, disorder, or condition results in an observable improvement in the patient's condition.

"Topical" used to define a mode of administration means that a material is administered by being applied to the skin or to an internal epithelial layer such as within the rectum, or colon or nasal or respiratory passage.

"Topically effective" means that a material applied to the skin or epithelial layer described above produces a desired pharmacological result either locally at the place of application or systemically as a result of transdermal passage of an active ingredient in the material.

"Oil-in-water formulation" refers to a formulation wherein a continuous water phase surrounds droplets of oil or lipid in an emulsion.

"Water-in-oil formulation" refers to a formulation wherein a continuous lipid or oil phase surrounds droplets of water in an emulsion.

"Non-aqueous formulation" refers to a formulation having less than 1%, and preferably less than 0.1%, by weight of water in the formulation. The term "non-aqueous" is also intended to include formulations having a negligible amount of water due to absorption of atmospheric moisture.

"Emulsifier" refers to a compound which is used to promote and maintain a stable mixture or dispersion (emulsion) of oil droplets in a water phase, or water droplets in an oil phase.

Emulsifiers are essentially surfactants. These surfactants can be ionic (cationic or anionic) or non-ionic and they can be used alone or in combination. Emulsifiers contemplated for use herein include, but are not limited to, cetearyl alcohol and sodium cetearyl sulfate, PEG-1000 monocetyl ether, glycol stearate, glyceryl stearate, cetyl alcohol, PEG-100 stearate, ceteareth-20, or quaternary ammonium salts such as alkyl trimethyl ammonium bromide, the polyol ester glycerol monostearate, potassium stearate, sodium lauryl sulfate, and ethoxylated fatty alcohols. Fatty acids like stearic acids may be included to regulate the consistency of the emulsion. Polymers such as carbomers can be included in small amounts to stabilize the emulsion.

Penetration enhancers are substances which enhance passage of topically-applied compounds into the stratum, corneum of the skin and therefrom into the epidermis and dermis. Examples include, but are not limited to: dimethylisosorbide, ethoxydiglycol, 1-dodecylazacycloheptan-2-one, propylene glycol, oleyl alcohol, polyoxyethylene ester, sorbitan mono-9-octadecenoate, poly(oxy-1,2-ethanediyl) and derivatives thereof, ethanol, glyceryl monoethyl ether, monoglycerides, isopropylmyristate, lauryl alcohol, lauric acid, lauryl lactate, terpinol, menthol, D-limonene, beta-cyclodextrin, DMSO (dimethyl sulfoxide), polysorbates, fatty acids (e.g., oleic), bile salts, N-methylpyrrolidone, polyglycosylated glycerides, 1-dodecylazacycloheptan-2-one (Azone®), Cyclopentadecalactone (CPE-215®), Alkyl-2-(N,N-disubstituted amino)-alkanoate ester (NexAct®), 2-(n-nonyl)-1,3-dioxolane (DEPA®), and penetration enhancers shown for example in U.S. Patents 3,909,816; 4,405,616; 4,801,586; 4,861,764; 4,886,783; 4,983,396; 5,118,845; and 5,196,410.

While the invention will now be described in connection with certain examples and embodiments so that aspects thereof may be more fully understood and appreciated, it is not intended that the invention be limited to these particular embodiments or examples. Thus the examples described herein which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of procedures as well as of the principles and conceptual aspects of the invention.

### Example 1

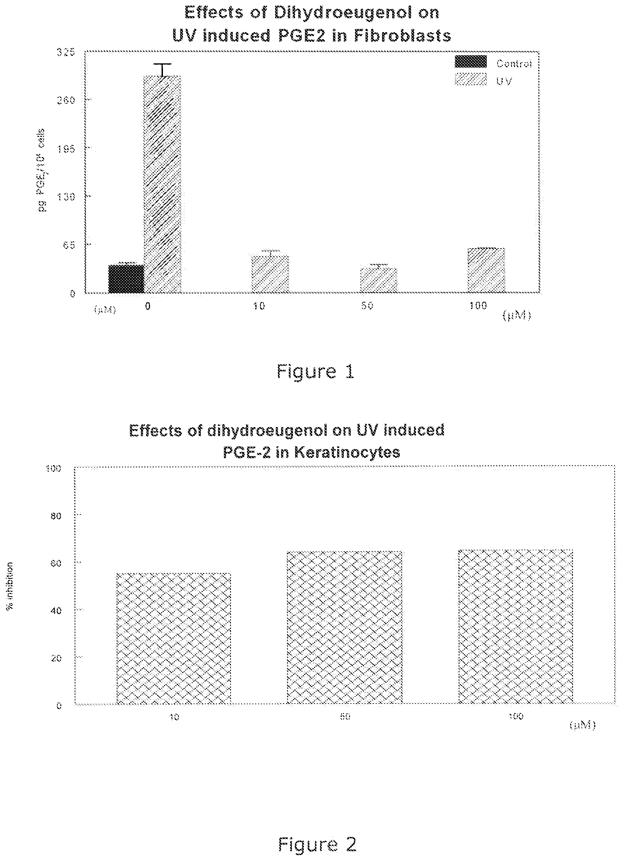

In one study, various concentrations of DHE were added to culture media which were placed on human fibroblast cell cultures (results shown in Fig. 1). Cells were either not induced or induced with UVB radiation to stimulate cytokine/PGE-2 production. At 24 hours after treatment, the cell culture medium was removed and assayed by ELISA methods for the expression of PGE-2, various cytokines and chemokines.

As is shown in Fig. 1, UV radiation (UVR) treatment of fibroblasts results in a 6 fold increase in PGE-2. When DHE at a concentration as low as 10 micromolar was put into the culture media after irradiation, it completely blocked the UVR induction of PGE-2.

When similar experiments were carried out with human keratinocytes, DHE was again found to markedly suppress the UVR induction of PGE-2, with a concentration of 50 uM inhibiting the production of PGE-2 by over 60% (results shown in Fig. 2). Since PGE-2 is the major inflammatory mediator responsible for sunburn these data indicate the efficacy of DHE in the treatment of sunburn. Further since PGE-2 is known to be a principal factor in the development of actinic keratosis and skin cancer, DHE can also be used in treating these conditions.

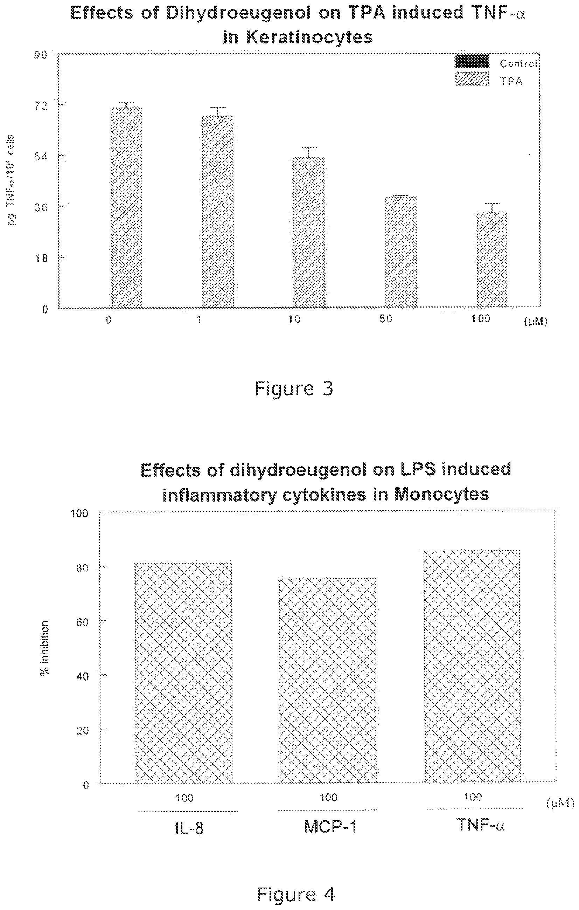

Inflammatory mediators produced in the skin contribute to the development and propagation of such diseases as rosacea, psoriasis and atopic dermatitis. While many inflammatory mediators are involved in these diseases, TNF-α is known to be a major cytokine involved in psoriasis. For atopic dermatitis, TNF-α, IL-8, and MCP-1 are important mediators of inflammation in these diseases. DHE is effective in suppressing the TPA-induced (TPA is tetradecanolyl phorbol ester) production of TNF-α by approximately 50% at a 100 micromolar concentration (see Fig. 3).

In monocytes (as shown in Fig. 4), dihydroeugenol can inhibit the production of the cytokine TNF-α as well as the chemokines, IL-8 and MCP-1 (monocyte chemotactic protein 1). Since these inflammatory mediators are critically important for the development of immune driven inflammatory diseases such as atopic dermatitis and psoriasis, the results indicate that DHE can be used to treat these diseases.

A summary of some of the inflammatory mediators blocked in keratinocytes, fibroblasts and monocytes is shown below in Tables 1, 2 and 3.

Table 1 shows the inhibitory effects of DHE and IE on production of several inflammatory mediators (PGE-2, IL-6, IL-8, and TNF-α) induced in keratinocytes by exposure to TPA and UV light. DHE and IE both have inhibitory effects on production of the inflammatory mediators in keratinocytes.

**TABLE 1. Percent Inhibition Of Inflammatory Mediators of Keratinocytes by DHE and IE**

| Active Compounds | DHE | IE |
|---|---|---|
| Concentration | 100 uM | 100 uM |
| | | |

| Keratinocytes (TPA) | | |
|---|---|---|
| PGE2 | 77% | 82% |
| IL-6 | 30% | 94% |
| IL-8 | 45% | 75% |
| TNF-α | 12% | 71% |
| | | |

| Keratinocytes (UV) | | |
|---|---|---|
| PGE2 | 68% | 76% |
| IL-6 | 30% | 93% |
| IL-8 | 40% | 76% |
| TNF-α | 31% | 60% |

Table 2 shows the inhibitory effects of DHE and IE on production of several inflammatory mediators (PGE-2, IL-6, and IL-8) induced in fibroblasts by exposure to IL-1 and UV light. DHE and IE both have inhibitory effects on the production of the inflammatory mediators in fibroblasts.

**TABLE 2. Percent Inhibition Of Inflammatory Mediators of Fibroblasts By DHE and IE**

| Active Compounds | DHE | IE |
|---|---|---|
| Concentration | 100 uM | 100 uM |
| | | |

| Fibroblasts (IL-1) | | |
|---|---|---|
| PGE-2 | 86% | 81% |
| IL-6 | 47% | 53% |
| IL-8 | 46% | 98% |
| | | |

| Fibroblasts (UV) | | |
|---|---|---|
| PGE-2 | 67% | 77% |
| IL-6 | 49% | 39% |
| IL-8 | 11% | 2% |

Table 3 shows the inhibitory effects of DHE and IE on production of several inflammatory mediators (MCP-1 (monocyte chemotactic protein-1), IL-12, TNF-α, and IL-8) induced in monocytes by LPS (lipopolysaccharide) and TNF-α. DHE and IE both have inhibitory effects on the production of the inflammatory mediators in monocytes.

**TABLE 3. Percent Inhibition Of Inflammatory Mediators of Monocytes By DHE and IE**

| Active Compounds | DHE | IE |
|---|---|---|
| Concentration | 100 uM | 100 uM |
| | | |

| Monocytes (LPS) | | |
|---|---|---|
| MCP-1 | 60% | 87% |
| IL-12 | 66% | 80% |
| TNF-α | 78% | 80% |
| IL-8 | 70% | 75% |
| | | |

| Monocytes (TNF-α) | | |
|---|---|---|
| MCP-1 | 46% | 82% |
| IL-8 | 72% | 78% |
| IL-12 | 98% | 100% |

### Example 2

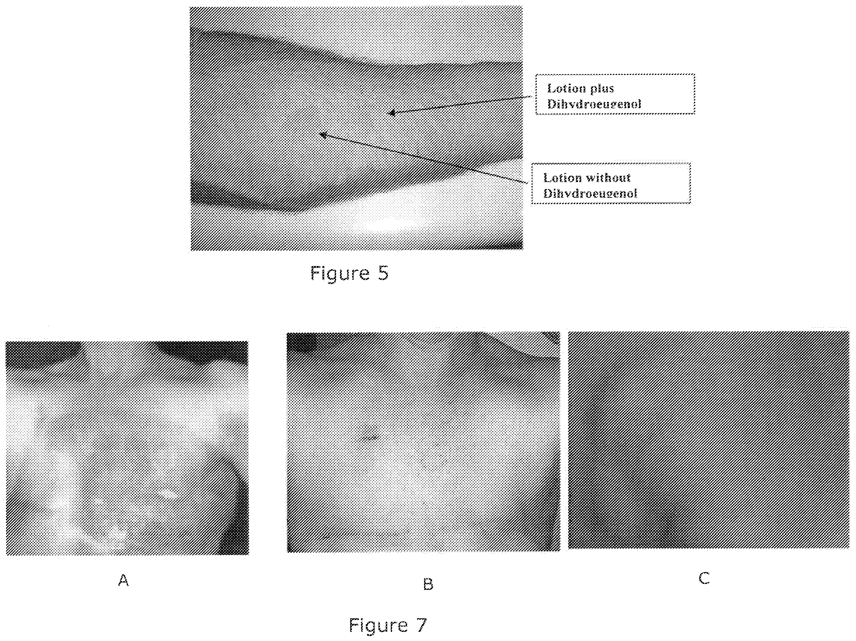

In one human clinical efficacy study the forearm of a clinical subject was irradiated at two sites with a dose of UVB radiation sufficient to induce erythema (Fig. 5). Immediately after the irradiation dose, but not before, one irradiated site was treated with a topical lotion containing 2% by weight of DHE while the other irradiated site was treated with the same topical composition without DHE (vehicle control). After 4 hours, pronounced erythema was visible at the site treated with the vehicle control while the site treated with 2% DHE lotion displayed only minimal erythema. This result was unexpected because stronger anti-inflammatory compounds (such as steroids) when used typically are unable to prevent UVB irradiation-induced erythema even though they are more effective than DHE in blocking inflammatory mediators (including PGE-2) in vitro. Therefore the actual mechanism of action of DHE in reducing UVB radiation induced erythema likely involves more than just inhibiting inflammatory cytokines and hormones such as PGE-2. The DHE-containing topical compositions not only are able to prevent induction of erythema but are also able to reverse erythema that is pre-existing.

### Example 3

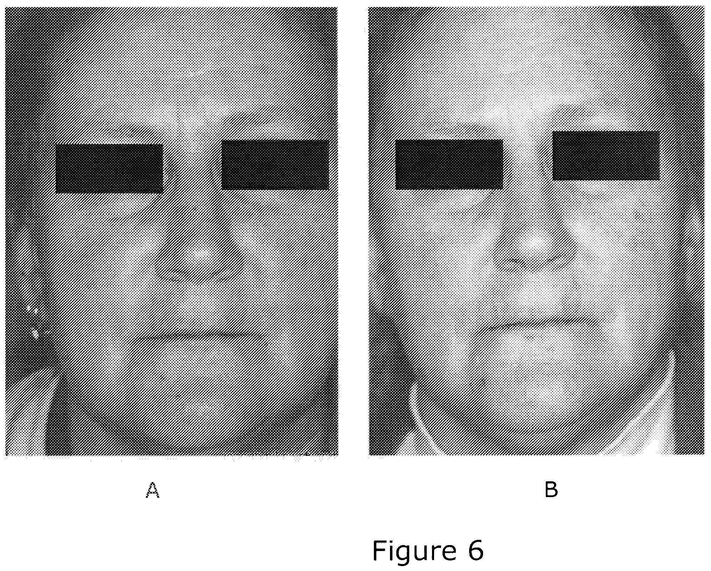

In addition to its ability to block radiation induced erythema (as shown in Example 2) another unexpected finding is that topically-applied DHE is effective in reducing the symptoms of rosacea, a disease whose etiology is for the most part not understood. Typical treatments for rosacea include topical metronidazole which is an antibiotic effective against bacteria and some parasites, and oral antibiotics. When topical DHE or a combination of DHE, IE and cinnamaldehyde was topically applied over 8-12 weeks to patients suffering from rosacea, the results revealed an overall improvement in their condition versus the same lotion base without DHE or DHE/IE/cinnamaldehyde. The results of this study are shown in Fig. 6A-B.

### Example 4

A clinical study carried out with cancer patients undergoing radiation therapy showed that topically-applied 2% DHE lotion (applied twice daily) can almost completely prevent the onset of radiation dermatitis. For example in a patient who underwent 35 radiation treatments for breast cancer and who was treated with a 2% DHE lotion (e.g., Formulation 5 shown below) daily during the 35 radiation treatments, there was no radiation damage after the 35 days of treatment or one month after the end of radiation treatment (see Fig. 7A-C).

### Example 5

For all aging studies, normal human fibroblast cell cultures were used. These cells normally produce collagen, elastin and hyaluronic acid. As the skin ages, the fibroblasts in the dermis lose their ability to produce these three key components of skin, and the skin consequently loses elasticity, thickness and smooth texture. In addition, as fibroblasts age, they increase the expression of certain enzymes that destroy collagen and elastin. There are about 13 of these enzymes, collectively referred to as MMPs (matrix metalloproteinases). MMP-1 is one of the principal MMPs responsible for collagen degradation. To determine the effects of DHE, IE and EV on collagen, elastin and hyaluronic acid, fibroblasts were treated with the compounds for 72 hours, mRNA was then isolated and analyzed for abundance by RT-PCR (Reverse transcriptase- Polymerase Chain Reaction). IE, DHE and EV were shown to stimulate collagen production in fibroblasts.

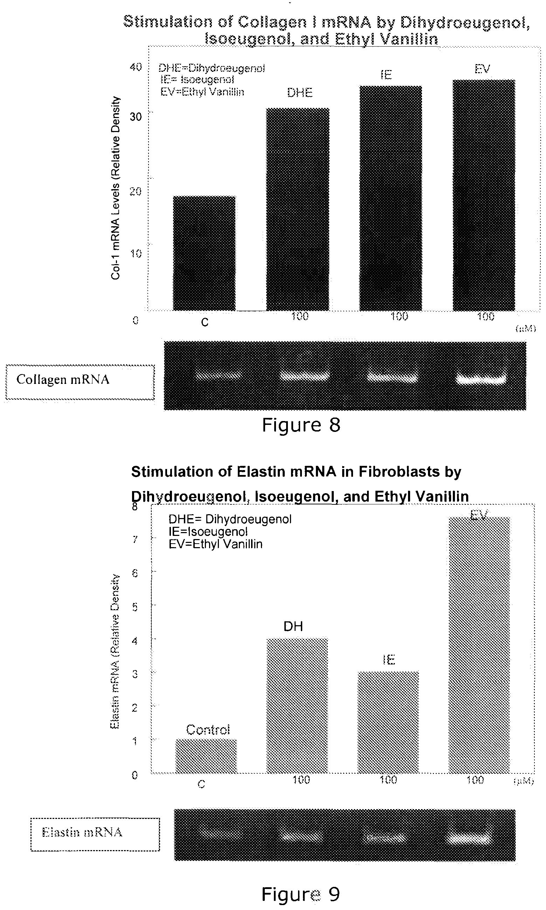

Fibroblast cell cultures were treated with either IE, DHE or EV for 72 hours at which time the amount of collagen mRNA present in the cells was determined. DHE, IE and EV each caused stimulation of collagen production in fibroblasts, as indicated by increased collagen mRNA production (Fig. 8).

In addition to stimulating collagen mRNA synthesis and collagen protein synthesis, DHE, IE and EV and salts, esters, ethers, and derivatives thereof can increase the level of elastin mRNA in human dermal fibroblasts as shown in Fig. 9. The image is from an electrophoresis gel of DNA amplified by PCR from fibroblast mRNA. This gel was analyzed and the density of each band quantified by image analysis software. The bar graph showing the densitometric analysis is shown above the image. DHE, IE and EV increase elastin levels in human dermal fibroblasts.

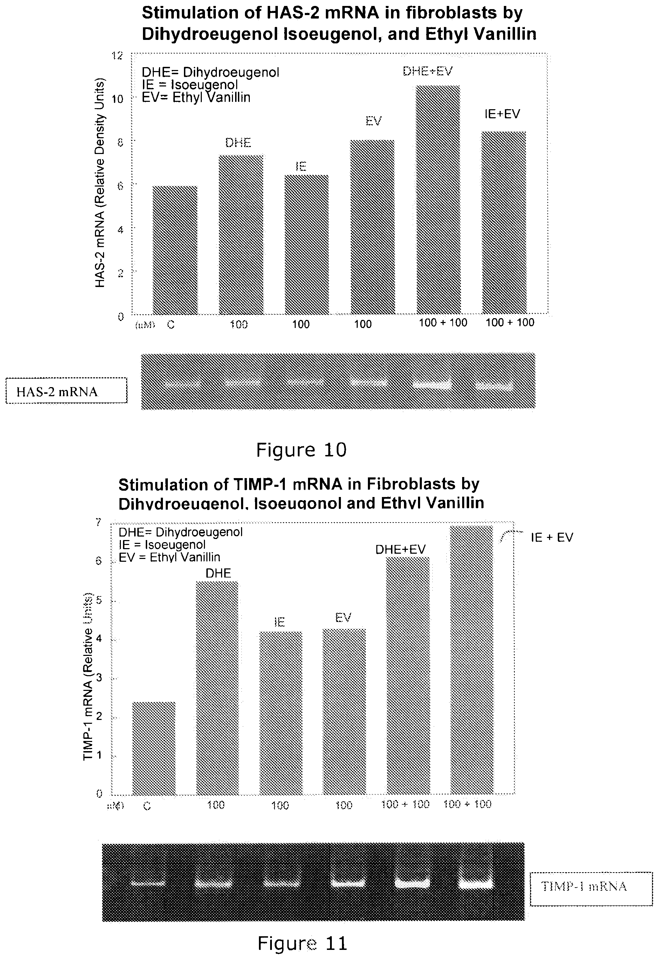

DHE, IE and EV, are also shown herein to stimulate mRNA for HAS-2. HAS-2 (Hyaluronic acid synthase-2) is the enzyme that manufactures hyaluronic acid (HA) in the skin. HA is a glycosaminoglycan important for maintaining moisture and suppleness in the skin. HA has a half-life of 24 hours and must therefore be continually replaced. As the skin ages, the production of HA decreases and this causes the skin to sag and become thin. It is shown herein that DHE either alone or in combination with EV can also stimulate the HAS-2 gene as shown in Fig. 10. In addition EV alone can stimulate HAS-2, but the combination of DHE and salts and esters thereof and EV synergistically works better than either one alone.

It has also been shown herein that TIMP production can be upregulated by DHE, IE and EV. Fibroblasts in the skin not only produce enzymes (MMPs) that destroy the skin matrix but they also produce proteins (Tissue Inhibitors of Metalloproteinases, i.e., TIMPS) that inhibit these enzymes. There are several TIMPs, but one that is most important for inhibiting MMPs is TIMP-1. It is also shown herein that DHE, IE and EV (and salts, esters, ethers and derivatives thereof) can significantly stimulate the production of TIMP-1, thereby providing the skin with protection against the matrix destroying activity of collagenase (MMP-1), the enzyme that TIMP-1 inhibits. Shown in Fig. 11 is PCR data showing the up-regulation of TIMP-1 mRNA by these compounds.

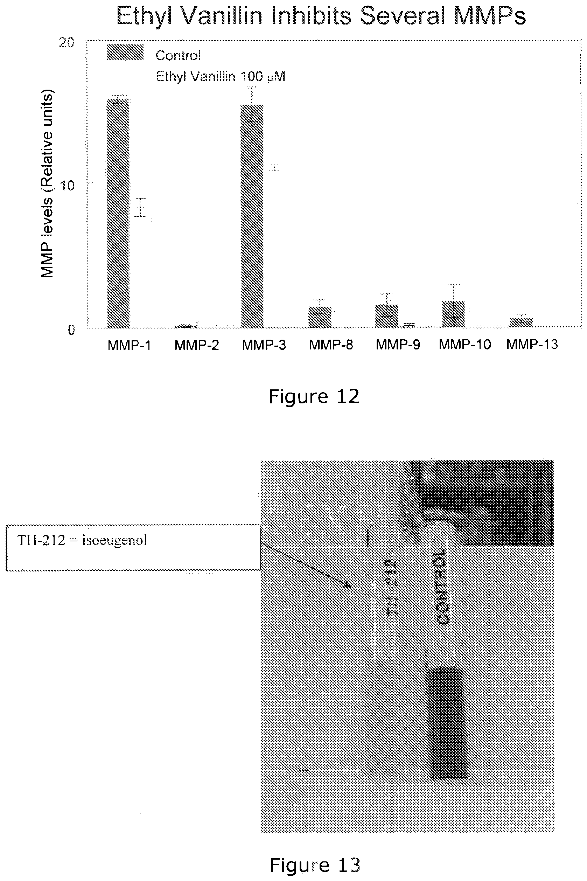

As mentioned above, as fibroblasts age they increase their production of MMP enzymes that destroy collagen and elastin, particularly collagenase, MMP-1. A protein array blotting technique was utilised to determine the effect of DHE, IE and EV on the protein abundance of these enzymes. As shown below, EV is able to reduce the level of several MMPs in human dermal fibroblasts, including MMP-1, MMP-3, MMP-8, MMP-9 MMP-10 and MMP-13 (Fig. 12). This remarkable effect makes this compound an ideal addition to an anti-aging product since MMP levels in aging skin are quite high.

### Example 6

### IE Inhibits Melanin Synthesis.

IE has been found to have a surprising and unexpected effect on blocking the synthesis of melanin catalyzed by tyrosinase. Two ml of a sodium phosphate buffer, pH 6.8 containing 0.2% L-DOPA (dihydroxyphenylalanine- a tyrosinase substrate) were placed in each of two test tubes. To one test tube was added 20 microliters of ethanol and to the other tube was added 20 microliters of a 1M stock of IE made up in ethanol (labeled TH-212). To start melanin synthesis from the L-DOPA substrate, 20 microliters of a tyrosinase preparation (0.5 mg/ml) was added to both tubes, the tubes were mixed and left at room temperature. The photograph in Fig. 13 was taken 2 hours after the start of the reaction. As can be seen in Fig. 13, melanin synthesis was almost completely blocked in the tube containing IE.

Without wishing to be bound by theory, it does not appear that IE is directly inhibiting tyrosinase, but rather is interfering with the post-tyrosinase steps required for melanin synthesis. IE does not appear to interfere with the tyrosinase mediated conversion of DOPA to dopaquinone or dopaquinone to dopachrome since the assay tube containing tyrosinase, DOPA and IE temporarily turns reddish. Since dopachrome is red, this indicates that IE is allowing tyrosinase to convert DOPA to dopaquinone. The conversion of dopaquinone to dopachrome is spontaneous and does not require tyrosinase. Isoeugenol may be converting dopachrome to some chemical entity that cannot proceed down the melanin synthesis pathway but remains as a colorless intermediate. Alternatively, IE may allow dopachrome to be converted to 5,6 dihydroxyindole, the next intermediate in the melanin pathway. It may then prevent the polymerization of 5,6 dihydroxyindole (a colorless intermediate) to melanin.

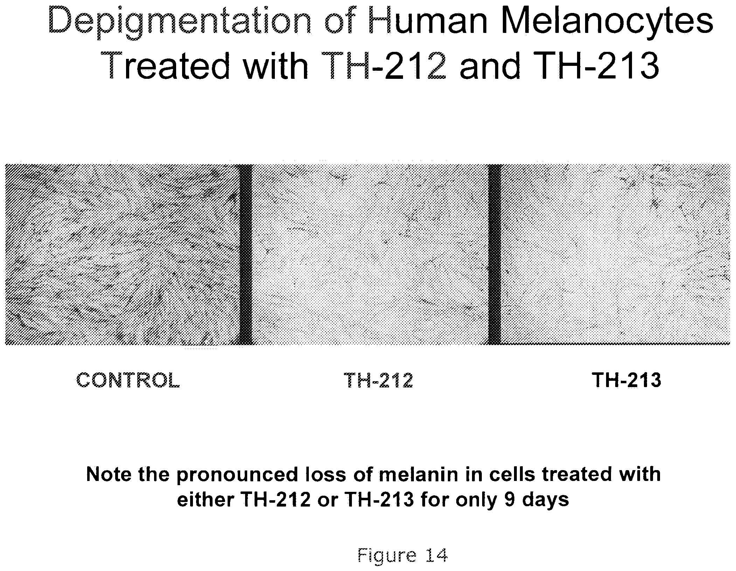

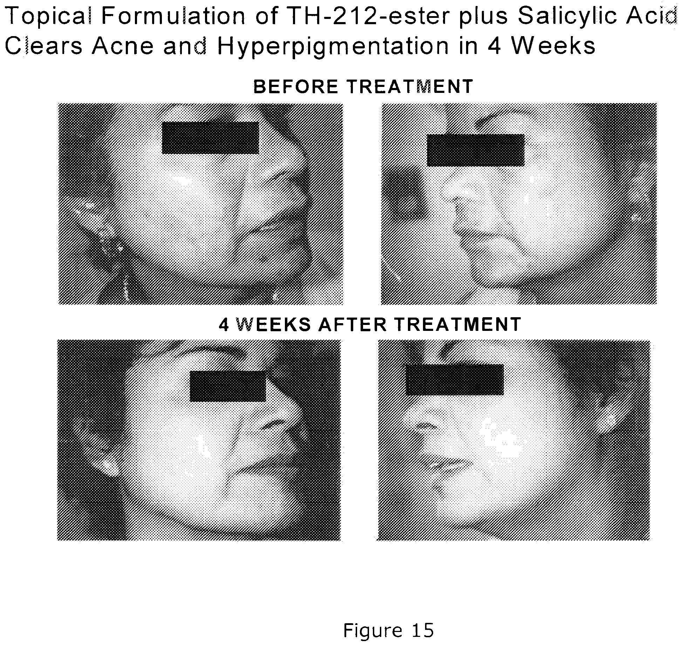

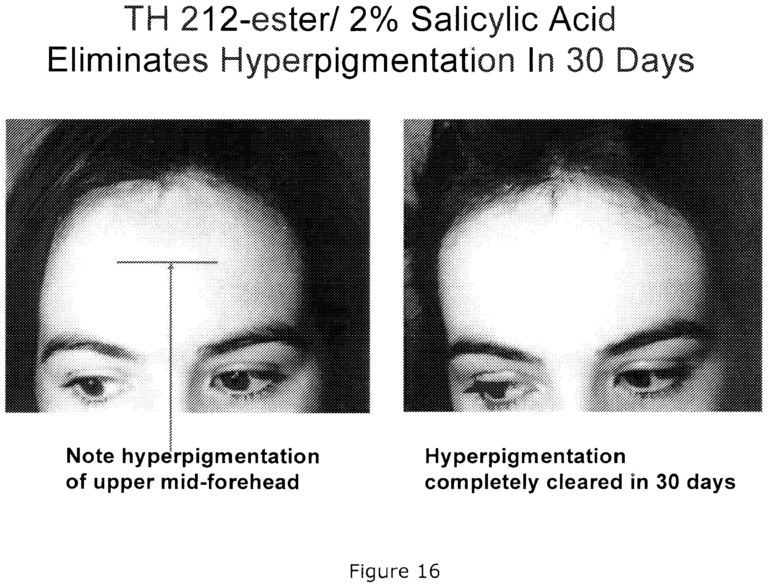

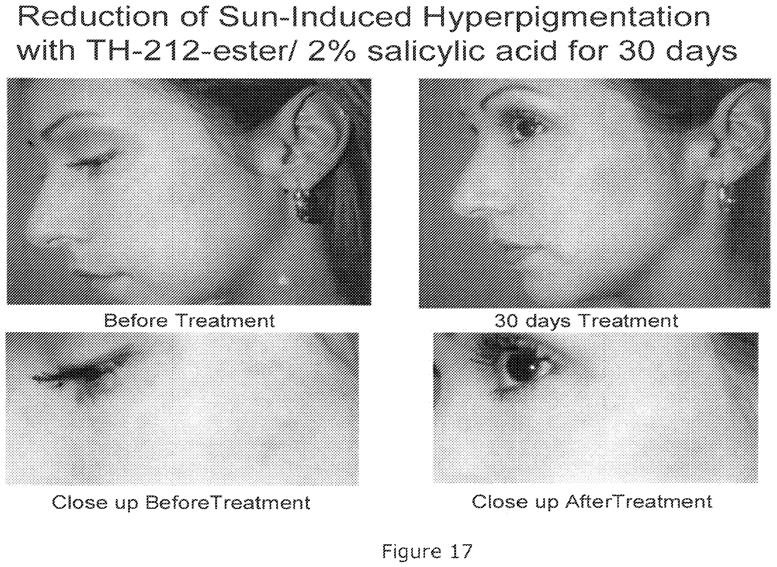

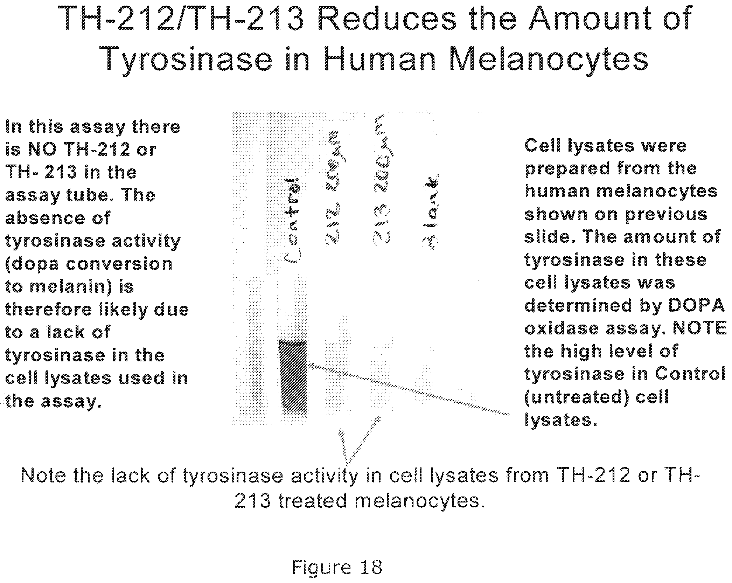

Figs. 14-17 show the skin-lightening effects of topically-applied formulations of isoeugenol and its ester isoeugenol acetate (a.k.a. isoeugenyl acetate) in the treatment of hyperpigmented areas in the skin, wherein a reduction of melanin pigment in the skin is caused by the topical application of the formulations. In addition to blocking a post-tyrosinase step in melanin synthesis, IE and IE acetate can inhibit the expression of the tyrosinase protein in human melanocytes. This inhibitory effect can be demonstrated by preparing cell extracts from melanocytes treated for 9 days with either IE or IE-acetate or left untreated. If the cell extracts contain tyrosinase when an aliquot of extract is placed in a tube containing L-DOPA (a tyrosinase substrate) in buffer, the enzyme will convert the DOPA to melanin. The melanin will appear brownish-black in the assay tube. If IE or IE-acetate has inhibited tyrosinase synthesis in melanocytes, then the extracts from these cells will contain no enzyme and will not be able to produce melanin from DOPA in the assay tube. As shown in Fig. 18 very little melanin was made in assay tubes that contained DOPA plus cell extracts from either IE or IE-acetate treated cells. An inhibitory effect of IE or IE-acetate on tyrosinase synthesis can also be demonstrated by the use of western immunoblots. In this assay, cell extracts from human melanocytes either left untreated or treated with 200 micromolar of either IE or isoeugenol acetate for 9 days are run on SDS polyacyrlamide electrophoresis gels to separate tyrosinase from other proteins. The tyrosinase in the gel is then blotted to a membrane and the tyrosinase detected by staining with a specific anti-tyrosinase antibody. The antibody-bound tyrosinase is then visualized by chemiluminescence. The results show that while untreated melanocytes have a high abundance of tyrosinase, the melanocytes treated for 9 days with either IE or isoeugenol acetate had almost no detectable tyrosinase present. This indicates that these compounds suppress the synthesis of the enzyme in human melanocytes.

Western immunoblots of tyrosinase abundance in human melanocyte extracts treated for 9 days with isoeugenol or isoeugenol acetate showed a considerable reduction in tyrosinase (as measured by RDU-relative densitometric units). The control showed approximately 7.25 RDU, while 200 µM concentrations of isoeugenol and isoeugenol acetate resulted in about 3 RDU and 3.75 RDU, respectively (data not shown).

Regardless of the mechanism by which IE or IE acetate or other compounds described herein have their effect, the inhibition of melanin production is essentially permanently blocked since test tubes containing DOPA, IE and tyrosinase fail to darken even after 2 weeks.

### Example 7

### Treatment of Psoriasis

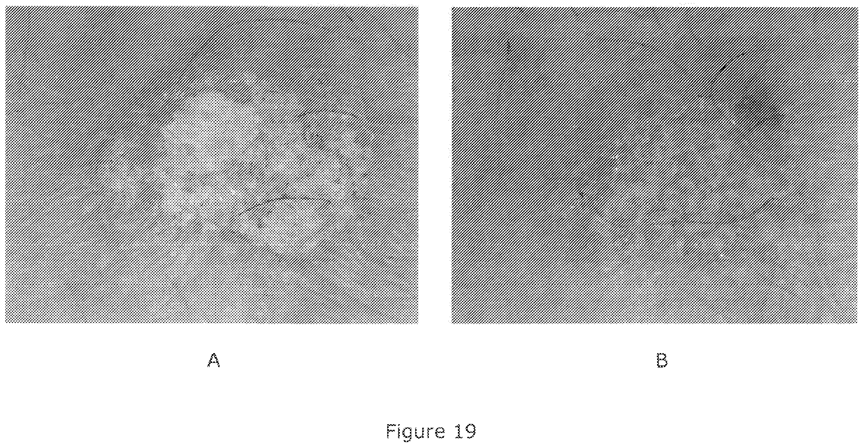

IE and DHE compositions (and salts, esters and ethers thereof) can be used to treat the scaly patches which occur in the skin of sufferers of psoriasis. Clinical treatments have shown significant reduction of scaling and inflammation after a 30-day course of treatment using the compositions of Examples 4-7. For example, Fig. 19 shows before (A) and after (B) pictures of a single psoriatic skin lesion after a 30 day treatment with a topical composition comprising DHE and IE.

### Example 8

### Effect of Water on Stability of EV.

A non-aqueous formulation of ethyl vanillin can be topically applied to the skin to stimulate fibroblast production of collagen, elastin, and TIMPs in the dermis and to reduce production of MMPs in the dermis, thereby inhibiting and counteracting the effects of aging of the skin and causing improved appearance thereof. Preferably the formulation comprises 0.1 to 5% of ethyl vanillin. The formulation may further be used to "re-model" scars in the skin wherein scars in the skin (including stretch marks and scarring due to acne) are treated so as to cause them to "disappear" i.e., to regain normal skin coloration and texture.

It has been shown herein that EV cannot be placed in a water-oil emulsion without the EV significantly decomposing within 24-48 hours. This surprising and unexpected finding meant that it was necessary to identify a carrier/vehicle system that would accommodate EV in a soluble form and at the same time maintain its chemical stability (defined herein as at least 95% of the ethyl vanillin remaining chemically intact for at least 3 months). In one formulation, EV is dissolved in caprylic/capric triglyceride or other fatty acid triglyceride and then mixed into a silicone fluid wherein the silicone is the primary (greatest percentage) component of the formulation. The cosmetic silicone fluid used in this embodiment accepts the caprylic/capric triglyceride and allows the EV to remain in solution. Because the formulation contains no water (or a negligible amount, such as less than 0.5%, due to absorption of atmospheric moisture) and no emulsifiers, the EV remains stable. Therefore formulations of EV in non-aqueous solutions may comprise at least 20% to 25% to 30% to 35% to 40% to 45% to 50%, or up to 60%, 70%, 80% or 90% silicone thereby explicitly excluding water-in-oil, or oil-in-water, emulsions. Additionally, besides caprylic capric triglyceride, EV can be solubilized in jojoba oil, sunflower oil or squalane or any other solubilizer which is accepted by the silicones.

For example, a water-in-oil formulation in which EV decomposes within 24 hours is:
water (62.8%), butanediol (5%), glycerin (4%), ethoxydiglycol (3%), glycereth-7 (2%), polysorbate 20 (0.2%), glyceryl stearate (and) PEG-100 stearate (4%), isocetyl stearate (3.5%), jojoba oil (3.5%), mineral oil (3%), isostearyl palmitate (3%), PEG-7 glyceryl cocoate (2%), isocetyl alcohol (2%), cetyl ricinoleate (1%), ethyl vanillin (1%).

### Pharmaceutical and Cosmetic Topical Compositions

The DHE-containing topical compositions are preferably administered in the form of pharmaceutical or cosmetic compositions. Such compositions can be prepared in a manner well known to those of ordinary skill in the pharmaceutical and cosmetic arts. As noted above, the DHE-containing topical compositions may further comprise cinnamaldehyde.

Generally, the DHE-containing topical compositions used in this invention are administered in a cosmetic amount or a therapeutically or cosmetically effective dose. The amount of the compound actually administered in a therapeutic setting may, typically be determined by a physician, such as a dermatologist in the light of the relevant circumstances, including the condition to be treated, the chosen route of administration, the actual compound administered, the age, weight, and response of the individual patient, the severity of the patient's symptoms, and the like. In cosmetic settings, the amount to be applied is selected to achieve a desired cosmetic effect.

The DHE or salt thereof (and where present IE, EV or salts, esters, ethers or derivatives thereof) is usually a minor component (or components) (from about 0.001 to about 20% by weight, or preferably from about 0.01 to about 10% or 0.1% to 5%, or 1.0% to 3%, by weight), with the remainder being various vehicles or carriers and processing aids helpful for forming the desired dosing form and for carrying the active agent into the epidermis. Cinnamaldehyde may be added in amounts of 0.001% to 0.5%, or more preferably 0.01% to 1%, or more preferably 0.05% to 0.5%. One preferred formulation comprises 0.4% DHE, 0.16% IE acetate and 0.04% EV and optimally 0.1% cinnamaldehyde.

Topical cosmetic and pharmaceutical dosing forms can include lotions, shampoos, soaks, gels, creams, ointments and pastes. Lotions commonly employ a water or alcohol base. Gels are semi-solid emulsions or suspensions. Creams generally contain a significant proportion of water in their base while ointments are commonly more oily.

Liquid forms, such as lotions suitable for topical administration or for cosmetic application, may include a suitable aqueous or non-aqueous vehicle with buffers, suspending and dispensing agents, thickeners, penetration enhancers, and the like. More solid forms such as creams or pastes or the like may include, for example, any of the following ingredients: water, oil, alcohol or grease as a substrate with surfactant, polymers such as polyethylene glycol, thickeners, solids and the like. Liquid or solid formulations may include enhanced delivery technologies such as liposomes, microsomes, microsponges and the like.

The above-described components for liquid, semisolid and solid topical compositions are merely representative. Other materials as well as processing techniques and the like are set forth in Remington's Pharmaceutical Sciences [*supra*].

When pharmaceutical compositions are to be administered transdermally they typically are employed as liquid solutions or as gels. In these settings the concentration of the DHE or salt thereof (and where present IE, EV or salts, esters, ethers or derivatives thereof) ranges individually or in combination from about 0.1% to about 20%, and preferably from about 0.1% to about 10%, and more particularly from 1% to 5%, of the composition with the remainder being aqueous mixed or nonaqueous vehicle, such as alcohols and the like, suspending agents, gelling agents, surfactant, and the like. Examples of suitable such materials are described below.

The DHE-containing topical composition can also be administered in sustained release transdermal forms or from transdermal sustained release drug delivery systems. A description of representative sustained release materials can be found in Remington's Pharmaceutical Sciences [*supra*].

The following examples illustrate representative cosmetic and pharmaceutical compositions used in this invention but is not limited thereto.

### Formulation 1- Lotion

In a preferred embodiment, DHE was formulated into a topical lotion with a 2% final concentration and tested for its ability to block a UVB-irradiation induced sunburn. Volunteers were irradiated with a UVB radiation source sufficient to produce a sunburn (approximately a 3 MED dose) and after irradiation 1 ml of a 2% topical DHE lotion was applied to one of two sites. Another irradiated site was left untreated. By 2-6 hours erythema was noted in the untreated UVB site while the side treated with the topical DHE lotion showed no erythema.

### Formulation 2- Liquid

A formulation of DHE (125 mg total) and xanthan gum (4 mg) are blended, passed through a No. 10 mesh U.S. sieve, and then mixed with a previously made solution of microcrystalline cellulose and sodium carboxymethyl cellulose (11:89, 50 mg) in a water/isopropanol (75:25) mixture. Sufficient water/isopropanol and salicylic acid to 2% by weight or a sufficient amount to maintain a pH of 3-6.5 are then added to produce a total volume of 5 mL.

### Formulation 3- Cream

A commercial mineral oil-water cold cream base is obtained. To 100 grams of this base, 1 gm of DHE (and where present IE, EV or salts, esters or derivatives thereof) is added as a fine powder or liquid with continuous mixing and stirring to suspend the powder in the base yielding a cosmetic or pharmaceutical composition.

In one embodiment, this composition includes the following: deionized water (55.6% by weight); niacinamide (2.0%); glycerin (4.0%); phenonip (1.0%); propylene glycol (5.0%); transcutol (3.2%); jojoba Oil (3.5%); isocetyl alcohol (2.0%); isocetyl stearate (3.5%); mineral oil (3.0%); dihydroeugenol (1.0 %); salicylic acid (2%); isostearyl palmitate (3.0%); PEG-7 glyceryl cocoate (2.0%); Glycereth-7 (2.0%); POLYSORBATE-20^{®} (0.2%); cetyl ricinoleate (1.0%); glyceryl stearate/PEG-100 stearate (4.0%); and SEPIGEL^{®} (2.0%).

### Formulation 4- Cream

Deionized water (56.4 % by weight); caffeine (1.0 %); butanediol (4.0 %); glycerin (1.0 %); phenonip (1.0 %); POLYSORBATE-20^{®} (0.2 %); niacinamide (2.0 %); arlacel (6.0 %); isocetyl stearate (3.5 %); cetyl ricinoleate (1.0 %); protaderm B (10.0 %); jojoba oil (3.5 %); stearyl alcohol (3.0 %); cetearyth 20 (0.4%); PEG-12 (3.0 %); dihydroeugenol (2.0 %); SEPIGEL™ (2.0%).

### Formulation 5- Cream

Water (44.4% by weight); niacinamide (2.0%); propylene glycol (3%); PEG-100 stearate (1%); ajidew (1%); glycerin (1%); EDTA (0.1%); carbopol (20%); squalene (2%); jojoba oil (2%); stearic acid (2%); glyceryl stearate (1%); cetyl alcohol (1.5%); vitamin E (1%); dimethicone (1%); caprylic/capric triglyceride (2%); dihydroeugenol (2%); petrolatum (1%); promulgen D (2%); PP2 (2%); glycol stearate (1%); dimethyl isosorbide-DMI (3%); and added after emulsion: germaben (1%) < 55°; and triethanolamine (1%).

### Ethyl Vanillin (EV) Formulations - Topical

The following are five non-aqueous formulations comprising EV which maintain EV in a chemically-stable condition which are provided as examples for illustrative purposes only.
1. Cyclomethicone (and) dimethiconol (20-90%), caprylic/capric triglyceride (1-20%), dimethicone (1-10%), ethyl vanillin (0.1-5%).
2. Cyclomethicone (and) dimethiconal (5-60%), cylcopentasiloxane (and) dimethicone crosspolymer (5-60%), caprylic/capric triglyceride (1-20%), dimethicone (1-10%), jojoba oil (1-10%), squalane (1-10%), ethyl vanillin (0.1-5%).
3. SD alcohol (5-50%), cetearyl octanoate (1-10%), vitamin E (0.5%), cyclomethicone (10-50%), PPG-26 oleate (1-10%), caprylic/capric triglyceride (1-10%), ethyl vanillin (0.1-5%).
4. PPG-15 Stearylether cyclomethicone (10-50%), Sunflower oil (10-50%), isopropyl alcohol (1-10%), isostearyl palmitate (1-10%), ethyl vanillin (0.1-5%).
5. Benzyl laurate (5-25%), PPG-10 butanediol (1-10%), mineral oil (10-70%), squalane (1-10%), caprylic/capric triglyceride (1-10%), ethyl vanillin (0.1-5%).

### 6. Utility and Dosing

The topical composition can be used in this invention topically to treat dermatological conditions such as actinic keratosis, acne, scarring, allergic contact dermatitis, atopic dermatitis, contact dermatitis, erythema (sunburn), hand eczema, itch, irritant contact dermatitis, psoriasis, seborrheic eczema (dermatitis), other eczemas, rosacea, alopecia areata, damage from radiation (radiation dermatitis) including UV radiation, IR radiation and any other ionizing radiation and the like, and other dermatological conditions described elsewhere herein.

Both cosmetic and pharmaceutical compositions can be used to treat and inhibit sunburn and to treat and prevent other forms of UV-induced inflammation and damage as well as damage from other forms of ionizing radiation.

In these uses, the dose levels or application levels can be expressed in terms of the amount of DHE (and where present IE, EV or salts, esters, ethers or derivatives thereof) delivered to the skin. For example, 1 to about 5 doses or applications per day, each containing from about 0.001 g to about 1 gram of each of DHE, IE and EV or salts, esters, ethers or derivatives thereof or combinations thereof can be used.

Alternatively, dose levels can be expressed in terms of the volume of topical composition administered. For example, 1 to about 5 doses or applications per day, each containing from about 1 to about 30 grams of topical composition containing alone or in combination from about 0.01% to about 10% by weight of DHE (and where present IE, EV or salts, esters, ethers or derivatives thereof) and especially from 0.02% to about 8% by weight or 0.1% to 5% by weight, or more preferably 1.0-4%.

When used in sun care products, such as suncare lotion, the concentration of DHE (and where present IE, EV or salts, esters, ethers or derivatives thereof) can be as set forth above and the product can be applied as needed based on the intensity and duration of sun exposure before, during, or after sun exposure.

In the case of transdermal administration to treat such inflammatory conditions, one can administer a quantity of topical composition used in the invention to a surface area of skin suitable to achieve an effective systemic bloodstream concentration of DHE (and where present IE, EV or salts, esters, ethers or derivatives thereof) eg of from about 0.5 µM to about 1000 µM and more preferably from about 1 µM to about 500 µM or other concentrations noted herein. It is preferred that the skin layer (epidermis and/or dermis) to be affected by the active agent maintain a concentration of active agent therein in a range of from 1 µM to 1000 µM, more preferably from 10 µM to 500 µM, more preferably from 50 µM to 300 µM, and still more preferably between 100 µM to 200 µM.

## Claims

1. Transdermal composition for use in the treatment of an inflammatory skin disorder, disease or condition in a subject, the transdermal composition comprising:
an active agent comprising dihydroeugenol or salts thereof, wherein the active agent is present in the range about 0.1% to about 10% by weight of the transdermal composition; and
the remainder of the transdermal composition being an aqueous, aqueous mixed or non-aqueous pharmaceutically-acceptable carrier or vehicle in which the active agent is suspended or dissolved, wherein the pharmaceutically-acceptable carrier or vehicle is effective in carrying or enabling passage of the active agent into the epidermis or dermis of the skin of the subject where the active agent has its effect on the inflammatory skin disorder, disease or condition and wherein the inflammatory skin disorder, disease, or condition of the subject is at least one of inflammation, rosacea, allergic contact dermatitis, irritant contact dermatitis, seborrheic dermatitis, radiation dermatitis, erythema, psoriasis, atopic dermatitis, eczemas, actinic keratosis, acne, scarring and alopecia areata.

2. Transdermal composition for use of claim 1 wherein the pharmaceutically-acceptable carrier or vehicle comprises a penetration enhancer.

3. Transdermal composition for use of either of claims 1 or 2 wherein the pharmaceutically-acceptable carrier or vehicle comprises a silicone fluid.

4. Transdermal composition for use of any one of claims 1-3 which is effective in percutaneous transmission such that the active agent can be maintained in the epidermis or dermis at a concentration in a range of from 10 µM to 500 µM.

5. Transdermal composition for use of any one of claims 1-3 wherein the active agent comprises 1% to 5% by weight of the transdermal composition.

## Patentansprüche

1. Transdermale Zusammensetzung zur Verwendung bei der Behandlung einer/s entzündlichen Hautstörung, -krankheit oder -zustands bei einem Patienten, wobei die transdermale Zusammensetzung umfasst:
ein aktives Mittel, umfassend Dihydroeugenol oder Salze davon, wobei das aktive Mittel vorhanden ist im Bereich von etwa 0,1% bis etwa 10%, bezogen auf das Gewicht der transdermalen Zusammensetzung; und
wobei der Rest der transdermalen Zusammensetzung ein wässriger/s, wässrig-gemischter/s oder nichtwässriger/s pharmazeutisch annehmbarer/s Träger oder Vehikel ist, in dem das aktive Mittel suspendiert oder aufgelöst ist, wobei der/das pharmazeutisch annehmbare Träger oder Vehikel wirksam ist bezüglich des Transportierens oder des Eintritts des aktiven Mittels in die Epidermis oder Dermis der Haut des Patienten, wobei das aktive Mittel seine Wirkung hat auf die entzündliche Hautstörung, -krankheit oder diesen -zustand und wobei die entzündliche Hautstörung, -krankheit oder dieser - zustand des Patienten mindestens einer ist von Entzündung, Rosacea, allergischer Kontaktdermatitis, reizender Kontaktdermatitis, seborrheischer Dermatitis, Strahlungsdermatitis, Erythem, Psoriasis, atopischer Dermatitis, Ekzemen, aktinischer Keratose, Akne, Vernarbungen und Alopecia areata.

2. Transdermale Zusammensetzung zur Verwendung nach Anspruch 1, wobei der/das pharmazeutisch annehmbare Träger oder Vehikel einen Penetrationsverstärker enthält.

3. Transdermale Zusammensetzung zur Verwendung nach einem der Ansprüche 1 oder 2, wobei der/das pharmazeutisch annehmbare Träger oder Vehikel ein Silikonfluid umfasst.

4. Transdermale Zusammensetzung zur Verwendung nach einem der Ansprüche 1-3, welche wirksam ist bei der perkutanen Transmission, so dass das aktive Mittel in der Epidermis oder Dermis gehalten werden kann, und zwar bei einer Konzentration im Bereich von 10 µM bis 500 µM.

5. Transdermale Zusammensetzung zur Verwendung nach einem der Ansprüche 1-3, wobei das aktive Mittel 1 Gew.-% bis 5 Gew.-% der transdermalen Zusammensetzung ausmacht.

## Revendications

1. Composition à usage transdermique pour une utilisation dans le traitement d'un trouble, d'une maladie ou d'un état cutané inflammatoire chez un sujet, la composition à usage transdermique comprenant :
l'agent actif comprenant du dihydroeugénol ou des sels de celui-ci, dans laquelle l'agent actif est présent à raison d'environ 0,1 % à environ 10 % en poids de la composition à usage transdermique ; et
le reste de la composition à usage transdermique étant un support ou véhicule pharmaceutiquement acceptable aqueux, aqueux mixte ou non aqueux, dans lequel l'agent actif est mis en suspension ou en solution, dans laquelle le support ou véhicule pharmaceutiquement acceptable est efficace pour transporter l'agent actif ou permettre son passage dans l'épiderme ou le derme de la peau du sujet où l'agent actif présente son effet sur le trouble, la maladie ou l'état cutané inflammatoire, et dans laquelle le trouble, la maladie ou l'état cutané inflammatoire du sujet est au moins l'un parmi une inflammation, une rosacée, une dermatite allergique de contact, une dermatite irritante de contact, une dermatite séborrhéique, une dermatite due à une irradiation, un érythème, un psoriasis, une dermatite atopique, un eczéma, une kératose actinique, l'acné, une cicatrisation et une alopécie en aires.

2. Composition à usage transdermique pour une utilisation selon la revendication 1, dans laquelle le support ou véhicule pharmaceutiquement acceptable comprend un amplificateur de pénétration.

3. Composition à usage transdermique pour une utilisation selon l'une ou l'autre des revendications 1 et 2, dans laquelle le support ou véhicule pharmaceutiquement acceptable comprend un fluide siliconé.

4. Composition à usage transdermique pour une utilisation selon l'une quelconque des revendications 1 à 3, qui est efficace en ce qui concerne la transmission percutanée de façon que l'agent actif puisse être maintenu dans l'épiderme ou le derme à une concentration située dans la plage allant de 10 µM à 500 µM.

5. Composition à usage transdermique pour une utilisation selon l'une quelconque des revendications 1 à 3, dans laquelle l'agent actif représente 1 % à 5 % en poids de la composition à usage transdermique.
